# EUROPEAN PATENT APPLICATION

(11) **EP 2 130 555 A1**
(43) Date of publication of application: **09.12.2009**
(21) Application number: 08010312.0
(22) Date of filing: 06.06.2008
(51) Int. Cl.: A61K 47/48, A61K 38/19, A61K 47/42, A61K 9/06

(54) **Stimuli-responsive hydrogel**

(71) Applicant: ETH Zurich, 8092 Zurich (CH)
(72) Inventor: Weber, Wilfried, 5200 Brugg/AG (CH); Fussenegger, Martin, 5506 Mägenwil/AG (CH)

(57) **Abstract**

The present invention relates to a hydrogel comprising a polymer, a first polypeptide and a polypeptide binding partner, wherein the polypeptide binding partner is a second polypeptide or a nucleic acid and wherein the interaction between the first polypeptide and the polypeptide binding partner stabilizes the hydrogel and is modulated by the addition of a modulating compound. A drug may be physically entrapped in the hydrogel, bound to the polymer forming the hydrogel structure, or bound to the first polypeptide or the polypeptide binding partner, and then be set free on addition of the modulating compound. Such a hydrogel comprising a drug may be injected into a patient, and drug release modulated by orally administering the modulating compound.

SEQUENCE LISTING

<110>  ETH Zürich

<120>  Stimuli-responsive hydrogel

<130>  P

<160>  2

<170>  PatentIn version 3.4

<210>  1
<211>  43
<212>  DNA
<213>  Artificial

<220>
<223>  gyr B probe

<400>  1
ggtacttgca catatgtcga attcttatga ctcctccagt atc                    43

<210>  2
<211>  44
<212>  DNA
<213>  Artificial

<220>
<223>  gyr B probe

<400>  2
ccagttacaa gcttatggtg atggtgatga tggccttcat agtg                   44

## Description

### Field of the invention

The invention relates to a hydrogel comprising a polymer, a polypeptide and a polypeptide binding partner, wherein the interaction of the polypeptide with its binding partner can be modulated by a third compound. This hydrogel is especially useful in drug delivery.

### Background of the invention

Stimuli-sensing hydrogels responsive to temperature, light, calcium, antigens, DNA and specific enzymes hold great promises as smart materials for drug delivery within the body (reviewed in Kopecek J., Eur J Pharm Sci 20, 1-16, 2043), for tissue engineering (Lutolf M. P. and Hubbell J. A., Nat Biotechnol 23, 47-55, 2005) or as (nano-) valves in microfluidic applications (Beebe D. J. et al., Nature 404, 588-90; 2000). Such materials commonly respond to triggers, which are difficult to apply in a patient background in the case of physical stimuli (e.g. light, temperature) or in the case of molecule-based stimuli due to stimulus concentrations hardly achievable in a physiologic background (e.g. antibody concentrations in the g/l range). In contrast, the mode of action for pharmaceutical substances is designed to occur within physiologic limits and therefore, hydrogels based on a pharmacologic mode of action are expected to show high compliance with future therapeutic applications.

### Summary of the invention

The present invention relates to a hydrogel comprising a polymer, a first polypeptide and a polypeptide binding partner, wherein the polypeptide binding partner is a second polypeptide or a nucleic acid and wherein the interaction between the first polypeptide and the polypeptide binding partner is modulated by the addition or withdrawal of a modulating compound.

In particular the invention relates to such a hydrogel, wherein the first polypeptide and the polypeptide binding partner are linked to the polymer, and wherein the interaction between the first polypeptide and the polypeptide binding partner stabilizes the hydrogel.

Either the first polypeptide or the polypeptide binding partner may be linked to the polymer, and the corresponding polypeptide binding partner or the first polypeptide, respectively, linked to a compound of interest, for example a drug. Alternatively, the compound of interest may be physically entrapped in the hydrogel, or bound to the polymer forming the hydrogel.

The interaction between the first polypeptide and the polypeptide binding partner is cleaved by the addition or withdrawal of a modulating compound.

The invention furthermore relates to a system of drug delivery comprising the hydrogel, wherein either the first polypeptide or the polypeptide binding partner are linked to the polymer, and the corresponding polypeptide binding partner or the first polypeptide, respectively, linked to a drug, and further comprising a compound cleaving the interaction between the first polypeptide and the polypeptide binding partner.

Likewise the invention relates to a system of drug delivery comprising the hydrogel, wherein the first polypeptide and/or the polypeptide binding partner are linked to the polymer and a drug is physically entrapped in the hydrogel structure stabilized by the interaction between the first polypeptide and the polypeptide binding partner, and further comprising a compound cleaving the interaction between the first polypeptide and the polypeptide binding partner thereby loosening the hydrogel structure to set free said drug. In a variation of this principle, the drug may be bound to the polymer forming the hydrogel. On addition of the compound cleaving the interaction between the first polypeptide and the polypeptide binding partner, the hydrogel breaks down and a drug-polymer complex is set free.

Accordingly, the invention relates also to a method of delivering a drug to a patient in need thereof, wherein a hydrogel is administered to the patient, wherein either the first polypeptide or the polypeptide binding partner are linked to the polymer, and the corresponding polypeptide binding partner or the first polypeptide, respectively, are linked to the drug, and after the hydrogel has reached its intended site of action the compound cleaving the interaction between the first polypeptide and the polypeptide binding partner is administered.

Likewise, the invention relates to a method of delivering a drug to a patient in need thereof, wherein a hydrogel is administered to the patient, wherein the first polypeptide and/or the polypeptide binding partner are linked to the polymer and a drug is physically entrapped in the hydrogel structure or bound to the polymer forming the hydrogel structure stabilized by the interaction between the first polypeptide and the polypeptide binding partner, and after the hydrogel has reached its intended site of action the compound cleaving the interaction between the first polypeptide and the polypeptide binding partner is administered thereby loosening the hydrogel structure to set free said drug, or the drug bound to the polymer, respectively.

### Brief description of the Figures

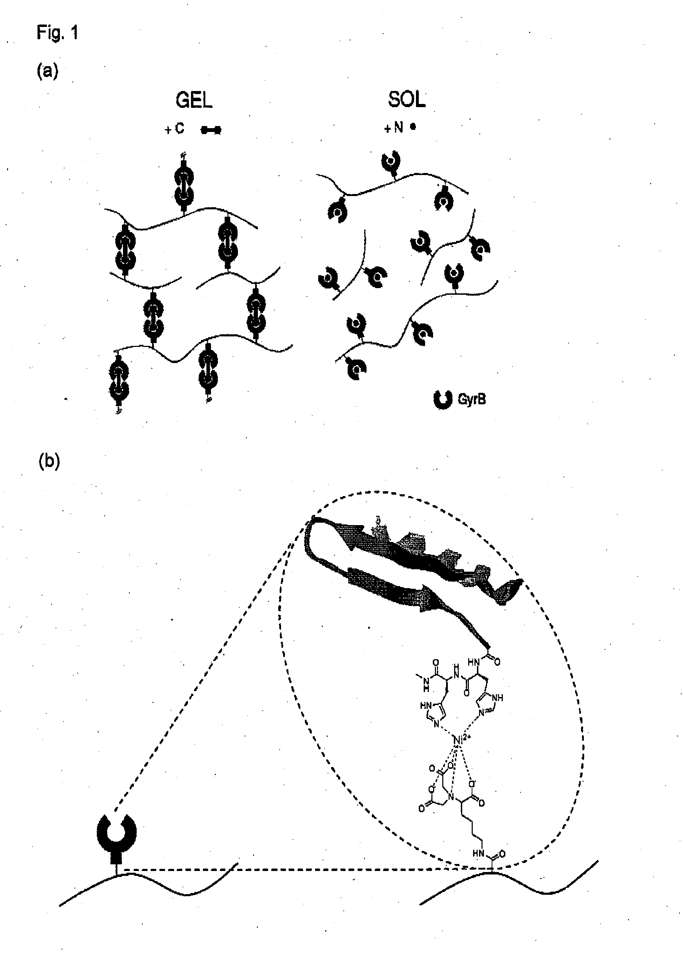

### Figure 1: Pharmacologically-triggered hydrogel

(a) Bacterial gyrase subunit B (GyrB) coupled to a polyacrylamide backbone is dimerized by coumermycin (+ C) resulting in gelation of the hydrogel. In the presence of novobiocin (+ N) GyrB is dissociated resulting in dissolution of the hydrogel.
(b) Coupling of proteins to the polyacrylamide backbone. Polyacrylamide is functionalized with nitrilotriacetic acid chelating a Ni²⁺ ion to which GyrB can bind via a hexahistidine sequence.

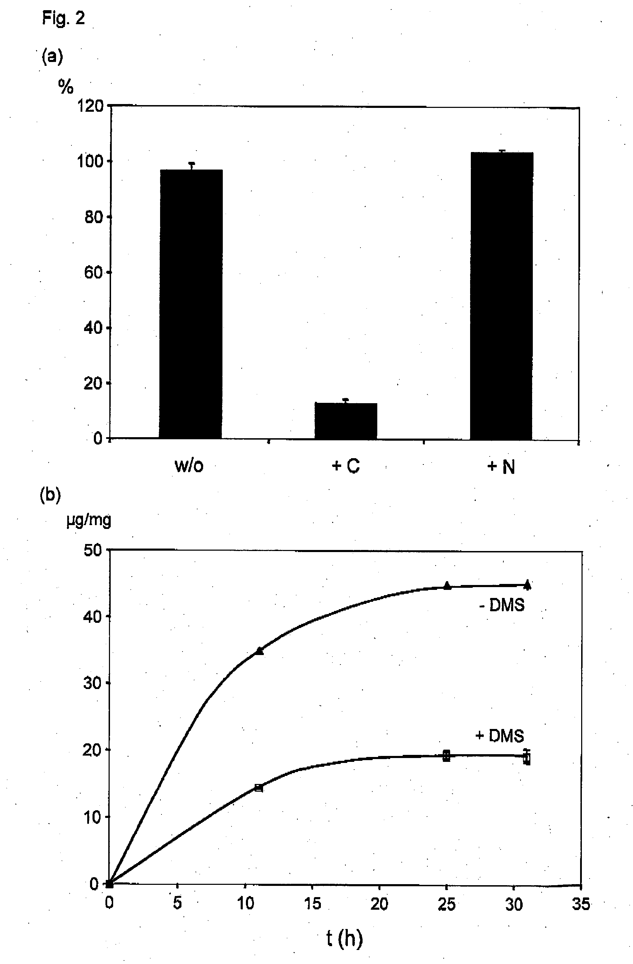

### Figure 2: Design of pharmacologically-controlled hydrogels

(a) Antibiotic-dependent hydrogel formation. Hexahistidine-tagged GyrB is incubated in the presence of coumermycin (+C, GyrB : coumermycin = 2 : 1, mol/mol), novobiocin (+N, GyrB : novobiocin = 1 : 10, mol/mol) or in the absence of any antibiotic (w/o). The GyrB complexes are mixed with Ni²⁺-charged poly(AAM-co-NTA-AAM) and the resulting viscous structures are incubated in PBS for 12 h prior to quantification of GyrB protein released into the buffer.
(b) GyrB dimerization-specific hydrogel. GyrB is dimerized by coumermycin (GyrB : coumermycin = 2:1) and further incubated in the presence (+ DMS) or absence (-DMS) of the amine-specific bifunctional crosslinker dimethyl suberimidate (DMS) for covalently stabilizing the GyrB dimers. GyrB dimers are mixed with Ni²⁺-charged poly(AAM-co-NTA-AAM) resulting in formation of the hydrogel. Following swelling over night in PBS, the hydrogels are placed in PBS containing 1 mM novobiocin and polymer dissolution is monitored by quantifying the released GyrB protein into the buffer.

### Figure 3: Adjustable pharmacologically triggered disintegration of the hydrogel

Hydrogels are incubated in PBS in the presence of different novobiocin concentrations (0 - 1 mM) and hydrogel disintegration is measured by quantification of GyrB released into the buffer.

### Figure 4: Human vascular endothelial growth factor 121 (VEGF₁₂₁) release

VEGF₁₂₁ is incorporated into the hydrogel and incubated in the presence of increasing novobiocin concentrations. VEGF₁₂₁ release into the buffer is followed over time.

### Figure 5: Novobiocin-induced swelling of the hydrogel

Hydrogels incorporating partially chemically crosslinked GyrB units are incubated in the presence or absence of 1 mM novobiocin while monitoring changes in polymer size.

### Detailed description of the invention

The present invention relates to a hydrogel comprising a polymer, a first polypeptide and a polypeptide binding partner, wherein the polypeptide binding partner is a second polypeptide or a nucleic acid and wherein the interaction between the first polypeptide and the polypeptide binding partner is modulated by the addition or withdrawal of a modulating compound.

Suitable polymers are, for example, poly-vinyl-based polymers like polyacrylamide, polyethylene glycol; poly-dimethyl-diallyl-ammonium chloride and N-(2-hydroxypropyl)-methacrylamide, polypeptides like fibrin, collagen and poly-L-lysine, and polycarbohydrates like alginate, cellulose and starch. Preferred polymers are, for example, polyethylene glycol, polyacrylamide and fibrin. Most preferred as the polymer is polyethylene glycol.

Pairs of a first polypeptide and a polypeptide binding partner, wherein the polypeptide binding partner is a second polypeptide, are for example, GyrB-GyrB (gyrase subunit B), FKBP-FRB (FK-binding protein - a domain (FRB) of the lipid kinase protein homologue FRAP (FKBP-rapamycin-associated protein)), F_{M}-F_{M} (F36M mutation of FK-binding protein), ToxT-ToxT (ToxT Protein of *V. cholerae*), DHFR-DHFR (dihydrofolate reductase), FKBP-FKBP (FK-binding protein), FKBP-Cyp (FK-binding protein - cyclophilin) and Cyp-Cyp (Cyclophilin). The corresponding modulating compound influencing the interaction between the polypeptides either by addition or withdrawal are, for example, coumarin antibiotics (for GyrB-GyrB), rapamycin or FK506 and derivatives (for FKBP-FRB and F_{M}), cyclosporins and derivatives (for Cyp), FK506 (for FKBP-FRB and F_{M}), virtstatin (for ToxT), and methotrexate and derivatives thereof (for DHFR-DHFR). Preferred as modulating compounds are small organic compounds, for example compounds of a molecular weight below 5000, in particular below 2000.

In a particular example, the first polypeptide and the polypeptide binding partner are the same compound having a tendency to form dimers. Particular examples of such dimerizing polypeptides are GyrB, F_{M}, ToxT, FKBP, and DHFR. The hydrogel comprising such dimerizing polypeptide may further contain a compound inducing dimerization. For example such a compound inducing dimerization are coumarin antibiotics, rapamycin and derivatives, virstatin, FK1012, and methotrexate and derivatives thereof. This dimerizing compound may fall under the definition of a modulating compound as set forth hereinabove or hereinbelow. Alternatively the modulating compound influencing the interaction between the first polypeptide and the polypeptide binding partner may be a modulating compound neutralizing the activity of the dimerizing compound, and by this neutralization lead to substantial reduction of the interaction of the dimerizing polypeptide. Such compounds which neutralize the dimerizing effect are, for example, the same compounds mentioned above to be dimerizing compounds when used in a substantial excess, or preferably other, different representatives of the same class of dimerizing compounds, e.g. the class of coumarin antibiotics, rapamycin and derivatives, and methotrexate and derivatives thereof, and also FK506.

Pairs of a first polypeptide and a polypeptide binding partner, wherein the polypeptide binding partner is a nucleic acid, are for example, E-ETR (MphR(A) protein and its operator ETR of *E. coli*), PIP-PIR (PIP protein of *Streptomyces pristinaespiralis* and its operator PIR), TetR-tetO (Tn10-derived tetracycline repressor TetR and its operator tetO), ArgR-argO (arginine-responsive repressor and its operator argO), ArsR-arsO (arsen-responsive repressor and its operator arsO), and HucR-hucO (uric acid-responsive repressor and its operator hucO). Other such pairs are the ones described by Ramos J. L. et al. (Microbiol Mol Biol Rev 69, 326-56, 2005), Martinez-Bueno M. et al. (Bioinformatics 20, 2787-91, 2004), and the ones that are listed in the database BacTregulators (http://www.bactregulators.org/). The corresponding modulating compounds influencing the interaction between the polypeptides either by addition or withdrawal are, for example, macrolide antibiotics (for E-ETR), streptogramin antibiotics (for PIP-PIR), tetracycline antibiotics (for TetR-tetO), arginine (for ArgR-argO), heavy metals (for ArsR-arsO), and uric acid (for HuOR-hucO).

Particular preferred pairs of a first polypeptide and a polypeptide binding partner, together with the corresponding modulating compound aminocoumarin antibiotics (e.g. coumermycin and novobiocin for GyrB-GyrB), rapamycin, FK506 and its derivatives AP21998 and AP22542 (for F_{M}-F_{M} and FKBP-FRB) are the combinations GyrB-GyrB, F_{M}-F_{M} and FKBP-FRB.

Most preferred are GyrB-GyrB and F_{M}-F_{M}.

In particular, the invention relates to such a hydrogel, wherein the first polypeptide and the polypeptide binding partner are linked to the polymer, and wherein the interaction between the first polypeptide and the polypeptide binding partner stabilizes the hydrogel. For example, the combination of GyrB with coumermycin is particularly useful for stabilizing a polymer. The polypeptide and its binging partner can be coupled to the polymer by specific linkers like, for example, chelate-forming entities like NTA and polyhistidine binding to a multivalent metal ion, peptide bonds, thiols coupled to maleimide or vinyl-sulfones, a halotag (Los G.V. et al., Methods Mol Biol. 356, 195-208, 2007), a SNAP-tag or a CLIP-tag (Gautier A. et al., Chem Biol. 15, 128-36, 2008) or by a transglutaminase reaction bond (Ehrbar M, et al., Biomaterials 29, 1720-9, 2008). Such hydrogels, are usually prepared by coupling the first polypeptide and the polypeptide binding partner to the polymer backbone mixing both reaction products and varying the concentration of the modulating compound in a way that the first polypeptide and the polypeptide binding partner interact with each other thereby forming a hydrogel. When changing the concentration of the modulating compound or adding a second modulating compound neutralizing the effect of the first one, the rigid structure is broken up and the hydrogel reverts to a substantially less rigid structure, e.g. a hydrosol.

For further stabilizing the hydrogel, additional cross-links can be introduced by chemically crosslinking the polymer backbone or crosslinking the first polypeptide with the polypeptide binding partner. Suitable crosslinkers are any homo- or heterofunctional compounds showing at least two sites for binding to another molecule like the ones described in Bioconjugate Techniques (2nd Edition by Greg T. Hermanson, Academic Press, 2008).

Additionally, semi-interpenetrating polymer networks (semi-IPN, meaning a polymer network of two or more polymers wherein at least one polymer is crosslinked and at least one polymer is not crosslinked, as described for example in Miyata T. et al., Nature 399, 766-769, 1999) containing the first polypeptide and the polypeptide binding partner are as well within the scope of the invention

Either the first polypeptide or the polypeptide binding partner may be linked to the polymer, and the corresponding polypeptide binding partner or the first polypeptide, respectively, linked to a compound of interest, for example a drug.

The drug may be any drug selected from the classes of cytostatic and cytotoxic drugs, antibiotics, antiviral drugs, anti-inflammatory drugs, growth factors and cytokines. Preferred drugs are those that cannot be administered orally, like polypeptide-based drugs.

In particular the drug is a drug which, to exert its full potential, has first to be transported to the site of action. Examples are monoclonal antibodies, growth factors and cytokines.

The drug may either be physically entrapped in the hydrogel structure or bound to the polymer forming the hydrogel, and might be released thereof as the free drug or as drug-polymer complex, respectively, by dissolution or swelling of the hydrogel induced by addition or withdrawal of the modulating compound, breaking the hydrogel structure stabilized by the interaction between polypeptide and the polypeptide binding partner. Alternatively, the drug is bound to the polypeptide binding partner, whereas the polypeptide binding partner is bound in the hydrogel to the first polypeptide. Addition or withdrawal of the modulating compound will cleave the interaction between the first polypeptides and the polypeptide binding partner thereby liberating the drug bound to the polypeptide binding partner. The reverse configuration, where the drug is bound to the first polypeptide and the polypeptide binding partner is immobilized in the hydrogel, is as well within the scope of this invention.

The invention furthermore relates to a system of drug delivery comprising the hydrogel wherein either the first polypeptide or the polypeptide binding partner are linked to the polymer, and the corresponding polypeptide binding partner or the first polypeptide, respectively, linked to a drug, and further comprising a compound cleaving the interaction between the first polypeptide and the polypeptide binding partner.

Preferred components of such a drug delivery system are those hydrogels mentioned above as being preferred, e.g. comprising a preferred polymer, preferred combinations of a first polypeptide and its polypeptide binding partner, and further the suitably adapted preferred modulating compound. Particular situations when such a hydrogel is preferably used are, for example, when the drug must be administered repeatedly or over a longer time frame. For example, the hydrogel may be applied by injection to the particular site of action, and the modulating compound might be applied orally, so that the modulating compound will diffuse to the site where the hydrogel has been injected, so that it will modulate the properties of the hydrogel (swelling or dissolution) and the liberation of the drug. Alternatively, hydrogels might be designed responsive to endogenous modulating compounds like uric acid so that a chance of the physiological concentrations of the endogenous compound will modify the properties of the gel and will modulate the liberation of the embedded drug.

Accordingly, the invention relates also to a method of delivering a drug to a patient in need thereof, wherein a hydrogel is administrered to a patient, wherein either the first polypeptide or the polypeptide binding partner are linked to the polymer, and the corresponding polypeptide binding partner or the first polypeptide, respectively, are linked to the drug, and after the hydrogel has reached its intended site of action the compound cleaving the interaction between the first polypeptide and the polypeptide binding partner is administered.

Likewise, the invention relates to a method of delivering a drug to a patient in need thereof, wherein a hydrogel is administered to the patient, wherein either the first polypeptide or the polypeptide binding partner are linked to the polymer and a drug is physically entrapped in the hydrogel structure or bound to the polymer forming the hydrogel structure stabilized by the interaction between polypeptide and the polypeptide binding partner, and after the hydrogel has reached its intended site of action the compound cleaving the interaction between the first polypeptide and the polypeptide binding partner is administered thereby loosening the hydrogel structure to set free said drug or drug-polymer complex, respectively.

Compared with a normal application of the drug to the patient the present method is much more convenient since the drug-comprising hydrogel must only be administred once by injection into the patient, and the drug can be released thereof on demand by taking an orally-available modulating compound. Thus repeated injections are replaced by one injection and some orally active compound in the form of a tablet, capsule, pill, or the like.

A particular hydrogel according to the invention is the antibiotic-responsive gel based on polyacrylamide grafted with bacterial gyrase subunit B (GyrB), which can be dimerized by the aminocoumarin antibiotic coumermycin, thereby resulting in gelation and three-dimensional stabilization of the hydrogel (Fig. 1a). Upon addition of the aminocoumarin novobiocin (Albamacin®), the interaction between GyrB and coumermycin is competitively inhibited, the three-dimensional structure is loosened and the hydrogel changes to the sol-state (Fig. 1a). In the particular example, the polymer backbone is polyacrylamide functionalized with nitrilotriacetic acid for chelating Ni²⁺ ions to bind hexahistidine-tagged (His₈) GyrB (Fig. 1 b). For construction of the polymer backbone, 2,2'-(5-acrylamido-1-carboxypentylazanediyl)diacetic acid (NTA-AAm) is synthesized, co-polymerized with acrylamide (AAm), and the NTA groups are charged with Ni²⁺. The resulting polymer poly(AAm-co-Ni²⁺-NTA-AAm) has a molecular mass of 42 kDa as judged from size exclusion chromatography with one NTA-AAm group per four acrylamide monomers as deduced from ¹H NMR analysis and reflecting the stoichiometry in synthesis.

The gene for *E. coli* gyrase subunit B (*gyrB*) is tagged with the coding sequence for six histidine residues. The coding region is placed under the control of the phage T₇-derived promoter and expressed in *E.coli* as a soluble cytoplasmatic protein. GyrB is purified via the hexahistidine tag using Ni²⁺-based affinity chromatography. Coumermycin-induced dimerization of genetically engineered GyrB is evaluated by incubating the protein in the presence or absence of coumermycin (GyrB : coumermycin = 2:1, mol/mol) with subsequent addition of the amine-specific bifunctional crosslinking agent dimethyl-suberimidate (DMS) and analysis of the complexes on denaturing polyacrylamide gel electrophoresis. In the absence of coumermycin, GyrB migrates predominantly at its predicted size of 27 kDa, whereas addition of the dimerizing antibiotic results in substantial dimer formation migrating at the predicted size of 54 kDa. In order to exclude that the remaining band migrating at 27 kDa in the presence of coumermycin results from inefficient antibiotic-mediated GyrB dimerization but rather from incomplete DMS-mediated covalent crosslinking, ultrafiltration experiments are performed. GyrB is incubated in the presence or absence of coumermycin and subjected to ultrafiltration using a 50 kDa molecular weight cut-off filter. GyrB in the absence of coumermycin passes the filter efficiently (54% of protein in filtrate), whereas only background GyrB levels can be detected in the filtrate, when coumermycin-dimerized GyrB is loaded (2.8% of protein in filtrate) indicating that coumermycin-mediated GyrB dimerization is quantitative.

Synthesis of coumermycin-crosslinked hydrogels is validated by incubating hexahistidine-tagged GyrB in the absence or presence of coumermycin or with a ten-fold molar excess of novobiocin. The protein is subsequently mixed with poly(AAm-co-Ni²⁺-NTA-AAm) at a ratio of one GyrB per 11 Ni²⁺ ions chelated in the polymer backbone. The solutions which all become viscous are incubated in PBS for 12 hours prior to quantification of GyrB-polymer complexes released into the buffer (Fig. 2a). In the absence of coumermycin or in the presence of novobiocin, the viscous structures are completely dissolved and GyrB is quantitatively retrieved in the buffer. However, in the presence of coumermycin, GyrB-polymer release is significantly reduced and a hydrogel can be observed thereby indicating the gelling effect of this dimerizing antibiotic (Fig. 2a). In order to demonstrate that the hydrogel formation is effectively due to coumermycin-mediated GyrB dimerization, hydrogels using coumermycin-dimerized GyrB as above or coumermycin-dimerized Gyr which had further been covalently crosslinked by DMS, are synthesized. Following swelling for 12 hours in PBS, the hydrogels are incubated in PBS containing 1 mM novobiocin and hydrogel dissolution is monitored by the release of GyrB-polymer complexes into the buffer (Fig. 2b). While coumermycin-crosslinked hydrogels are dissolved after 11 hours in the presence of novobiocin, hydrogels with DMS-crosslinked GyrB (+ DMS) are stable for the observation period of 31 hours (Fig. 2b). This observation confirms that the hydrogel is effectively formed by coumermycin-mediated dimerization of GyrB, which can be reversed by excess novobiocin. Specificity is further demonstrated by addition of antibiotics from other classes (e.g. β-lactams, macrolides), where no impact on gel dissolution can be observed.

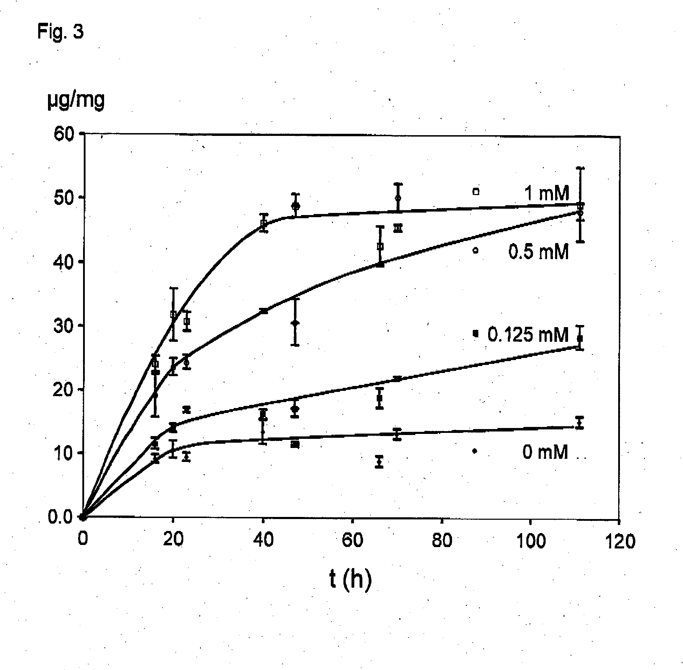

Pharmacologically-triggered hydrogel formation and dissolution opens new perspectives for optimal delivery of protein-based pharmaceuticals within the body, provided that the dissolution kinetics of the hydrogel and the release properties of the biopharmaceutical can optimally be adjusted into the therapeutic window. In order to investigate adjustable hydrogel characteristics, coumermycin-dimerized hydrogel is incubated in the presence of increasing novobiocin concentrations, and gel dissolution followed by quantification of released GyrB-polymer complexes (Fig. 3). In the presence of 1 mM novobiocin, the hydrogel dissolves rapidly whereas lower novobiocin concentrations correlate with slower hydrogel dissolution and slower GyrB release demonstrating adjustable dissolution and release kinetics (Fig. 3). The hydrogel is stable for 24 days in the absence of novobiocin.

Addition of 1 mM novobiocin at day 24 results in dissolution of the hydrogel until day 26 demonstrating the long-term functionality of the hydrogel.

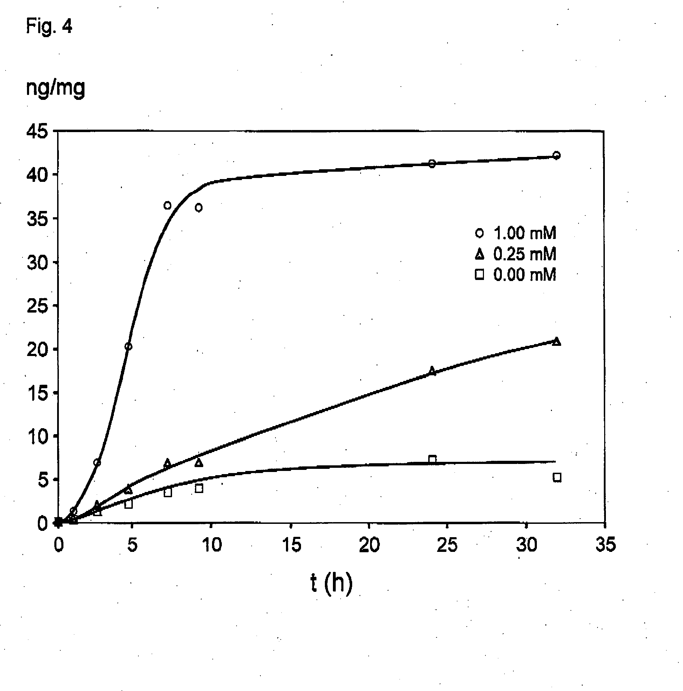

In order to demonstrate pharmacologically-triggered release of a therapeutic protein from the stimuli-sensing hydrogel, human vascular endothelial growth factors 121 (VEGF₁₂₁). VEGF₁₂₁ is incorporated into the hydrogel (GyrB : VEGF₁₂₁ = 1000:1, mol/mol) and incubated in the presence of increasing novobiocin concentrations (Fig. 4). In the presence of 1 mM novobiocin, VEGF₁₂₁ is completely released within 10 hours, while only background VEGF₁₂₁ levels are observed in the absence of the stimulus. At intermediate novobiocin concentrations (0.25 mM) VEGF₁₂₁ release kinetics are slower, thereby demonstrating the trigger-adjustable growth factor release characteristics.

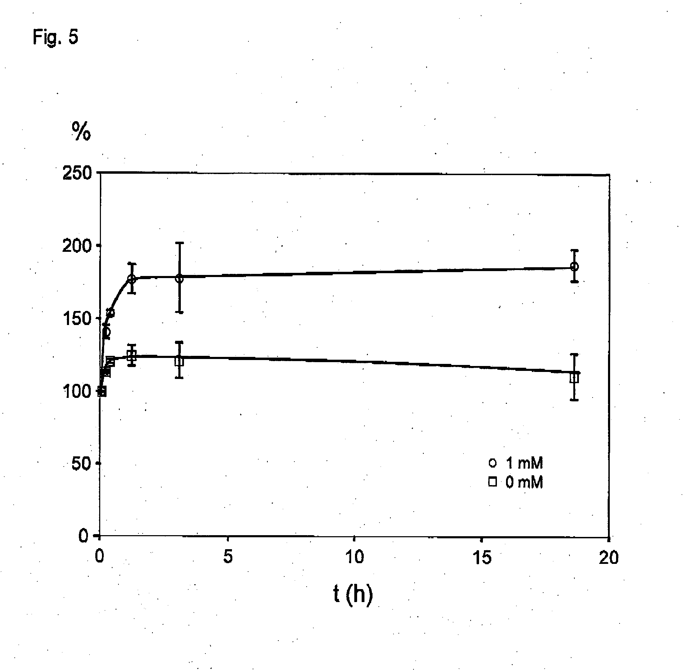

The GyrB-based system can as well be used to design hydrogels that swell in the presence of novobiocin. Therefore, hydrogels are prepared as described above with the modification that the coumermycin-dimerized GyrB molecules are chemically crosslinked by equimolar amounts of dimethyl suberimidate. When such gels are incubated in the presence of novobiocin, swelling can be observed (Fig. 5).

### Examples

### Example 1: Production of hexahistidine-tagged GyrB

Bacterial gyrase subunit B gene (*gyrB*) is amplified from *E. coli* DH5α chromosomal DNA using oligonucleotides OWW866 (5'-ggtacttgcacatatgtcgaattcttatgactcctccagtatc-3', SEQ ID NO:1) and OWW867 (5'-ccagttacaagcttatggtgatggtgatgatggccttcatagtg-3', SEQ ID NO:2) and ligated (*Nd*el/*Hin*dIII) into pWW301 (Weber C. C. et al., Biotechnol Bioeng 89, 9-17, 2005) thereby placing *gyrB* under the control of the phage T₇ promoter. pWW873 is transformed into *E. coli* BL21 STAR™ (DE3) (Invitrogen, Carlsbad, CA, cat. no. C601003) and protein production is induced at OD₈₀₀ = 1 by 1 mM IPTG for 3 h at 37°C. The cell pellet is resuspended in PBS (40 ml per 1000 ml initial culture volume, 50 mM NaH₂PO₄, 300 mM NaCl, 10 mM imidazole, pH 8.0), disrupted using a French press (Thermo Fisher Scientific, Waltham, MA), and cell debris are eliminated by centrifugation at 15,000 x g for 20 min. The cleared cell lysate is loaded onto an NTA-agarose Superflow column (Qiagen, Hilden, Germany, cat. no. 30210), which is subsequently washed with 10 column volumes PBS, 10 column volumes wash buffer (50 mM NaH₂PO₄, 300 mM NaCl, 20 mM imidazole, pH 8.0) and eluted with 2 column volumes elution buffer (50 mM NaH₂PO₄, 300 mM NaCl, 250 mM imidazole, pH 8.0). The elution buffer is exchanged to PBS by ultrafiltration (10 kDa MWCO, Sartorius, Göttingen, Germany, cat. no. VS0202), and GyrB is concentrated to 80 mg/ml.

### Example 2: Production of VEGF_{121.}

The expression vector pRSET-VEGF₁₂₁ (Ehrbar M. et al., Circ Res 94, 1124-32, 2004) for hexahistidine-tagged human vascular endothelial growth factor 121 (VEGF₁₂₁) is transformed in *E_{.} coli* BL21 STAR™ (DE3), and protein production is induced at OD₆₀₀ = 0.8 by addition of 1 mM IPTG for 4 h at 37°C. The cell pellet is resuspended in 50 mM Tris/HCl, 2 mM EDTA, pH 8.0 (100 ml per 1000 ml initial culture volume), the cells are disrupted using a French press and the inclusion bodies are pelleted by centrifugation at 15'000 x g for 20 min at 4°C. Inclusion bodies are dissolved over night at 4°C in 6 M urea, 500 mM NaCl, 5 mM imidazole, 20 mM Tris/HCl, pH 7.9 and separated from the cell debris by centrifugation (15'000 x g, 30 min, 4°C). The supernatant is loaded onto an NTA-agarose Superflow column following washing (15 column volumes 6 M urea, 500 mM NaCl, 20 mM imidazole, 20 mM Tris/HCl, pH 7.9) and elution (6 M urea, 500 mM NaCl, 250 mM imidazole, 20 mM Tris/HCl, pH 7.9). The eluate is incubated for 30 min at 22°C with 2 mM DTT and 2 mM EDTA to reduce disulfide bonds followed by a three step dialysis (3.5 kDa MWCO, Pierce, Rockford, IL, cat. no. 68035): 2 x 1 h in 4 M urea, 1 mM EDTA, 20 mM Tris/HCl, pH 7.5; 2 x 1 h in 3 M urea, 1 mM EDTA, 20 mM Tris/HCl, pH 7.5; 1 h in 2 M urea, 150 mM NaCl, 20 mM Tris/HCl, pH 7.5 and over night in 2 M urea, 154 mM NaCl, 20 mM Tris/HCl, pH 7.5. Purified VEGF₁₂₁ is stored at -80°C. VEGF₁₂₁ is quantified using a sandwich ELISA (Peprotech, Hamburg, Germany, cat. no. 900-K10).

### Example 3: Characterization of GyrB

Concentration of GyrB is analyzed by the Bradford method (Biorad, München, Germany, cat. no. 500-0006) using BSA as standard. For SDS-PAGE analysis, 12% reducing gels are used with subsequent Coomassie staining. Antibiotic-triggered dimerization of GyrB is analyzed by incubating GyrB in the presence coumermycin A1 (GyrB: coumermycin = 2 : 1, mol/mol; Sigma, St. Louis, MO, cat. no. C9270) for 30 min at room temperature following covalent crosslinking of the dimers by addition of dimethyl suberimidate x 2 HCl (DMS, SigmaAldrich, St. Louis, MO, cat. no. 179523) at a 10-fold molar excess with respect to GyrB for 60 min at room temperature. The dimers are analyzed on SDS-PAGE. For ultrafiltration studies of the coumermycin-induced dimerization, 100 nmol GyrB in 1 ml PBS are incubated with or without 50 nmol coumermycin for 30 min at room temperature. Following addition of 4 mi PBS, the solution is subjected to ultrafiltration (50 kDa MWCO, Filtron, Northborough, MA, cat. no. OD050C36) at 5000 x g for 45 min. The retentate (1 ml) is diluted with 5 ml PBS and ultrafiltrated again prior to quantification of the GyrB concentration in the pooled filtrate and retentate.

### Example 4: Synthesis of 2,2'-(5-acrylamido-1-carboxypentylazanediyl)diacetic acid (NTA-AAm)

3.3 mmol acryloyl chloride (ABCR, Karlsruhe, Germany, cat. no. AB172729) dissolved in 15 ml toluene are dropwise added during 4 h to an ice-cooled solution of 3 mmol N,N-bis(carboxymethyl)-L-lysine (Fluka, Buchs, Switzerland, cat. no. 14580) dissolved in 27 ml 0.44 M NaOH. The toluene is evaporated in *vacuo* and sodium ions are removed with Dowex® 50WX8 (Acros, Geel, Belgium, cat. no. 335351000) prior to lyophilization resulting in a viscous oil (yield: 50%).

### Example 5: Synthesis of poly(AAm-co-NTA-AAm)

1.5 mmol NTA-AAm and 6.4 mmol acrylamide (AAm, Pharmacia Biotech, Uppsala, Sweden, cat. no. 17-1300-01) are dissolved in 48 ml 50 mM Tris/HCl, pH 8.5 under nitrogen; and polymerization is initiated by addition of 150 µl ammonium peroxodisulphate (APS, 10%, w/v) and 24 µl N,N,N',N'-tetramethylethylenediamine (TEMED) for 20 h at room temperature. The polymer is concentrated to 20 ml *in vacuo* and subsequently dialyzed twice (3.5 kDa MWCO, Pierce, Rockford, IL, cat. no. 68035) against 2 I H₂O for 12 h to eliminate salts and toxic low molecular weight compounds like residual acrylamide. The obtained molar ratio of AAm to NTA-AAm is 4 to 1 as determined by ¹H NMR (Avance 500 Bruker BioSpin AG Fällanden, Switzerland). The dialysate is supplemented with 3.5 mmol NiSO₄ and dialyzed twice against 0.5x PBS for 12 h and twice against 0.1x PBS for 12 h. The Ni²⁺-charged polymer is concentrated 10-fold *in vacuo* resulting in a 6% (w/v) solution. The size of Ni²⁺-charged poly(AAm-co-NTA-AAm) is analyzed by gel permeation chromatography on a Shodex OHpak SB-806 HQ (8.0 mm x 300 mm, Showa Denko, Kawasaki, Japan) column using PBS as mobile phase at a flow rate of 0.5 ml/min (Waters 2796 Alliance Bio, Waters AG, Baden, Switzerland). Detection is performed at 280 nm and 390 nm using a Waters 2487 UV-detector. As size standards, poly(styrenesulfonic acid sodium salt) (Fluka, Buchs, Switzerland) is used.

### Example 6: Hydrogel formation and characterization

Purified GyrB (80 mg/ml) in PBS is mixed with coumermycin (50 mg/ml in DMSO) at a molar ratio of GyrB : coumermycin = 2:1 and incubated for 30 min at room temperature. Dimerized GyrB is subsequently added to 4.5 µl poly(AAm-co-NTA-AAM) (as 6% w/v solution in PBS) per mg GyrB and mixed by gently stirring. The hydrogel forms immediately and is incubated at 4°C in a humidified atmosphere for 20 h prior to incubating the hydrogel for 12 h in PBS. For investigation of trigger-inducible hydrogel dissolution, the gel is incubated in PBS in the presence of different novobiocin (Fluka, cat. no, 74675) concentrations and the dissolution is monitored optically (GelJet Imager 2004, Intas, Göttingen, Germany) and by quantification of GyrB release into the buffer using the Bradford method. Error bars represent the standard deviation from three experiments.

### Example 7: Hydrogel formation and characterization with additional crosslinks

Purified GyrB (80 mg/ml) in PBS is mixed with coumermycin (50 mg/ml in DMSO) at a molar ratio of GyrB : coumermycin = 2:1 and incubated for 30 min at room temperature. Dimerized GyrB is subsequently incubated with an equimolar amount of dimethyl suberimidate and incubated at room temperature for 60 min. Dimerized and crosslinked GyrB is subsequently added to 4.5 µl poly(AAm-co-NTA-AAM) (as 6% w/v solution in PBS) per mg GyrB and mixed by gently stirring. The hydrogel forms immediately and is incubated at 4°C in a humidified atmosphere for 20 h prior to incubating the hydrogel for 20 h in PBS or in PBS supplemented with 1 mM novobiocin. Swelling of the gel is monitored optically (Fig. 5). Error bars represent the standard deviation from three experiments.

## Claims

1. A hydrogel comprising a polymer, a first polypeptide and a polypeptide binding partner, wherein the polypeptide binding partner is a second polypeptide or a nucleic acid and wherein the interaction between the first polypeptide and the polypeptide binding partner is modulated by the addition or withdrawal of a modulating compound.

2. The hydrogel of claim 1, wherein the first polypeptide and the polypeptide binding partner are linked to the polymer, and wherein the interaction between the first polypeptide and the polypeptide binding partner stabilizes the hydrogel.

3. The hydrogel of claim 1, wherein the first polypeptide is linked to the polymer and the polypeptide binding partner is linked to a compound of interest.

4. The hydrogel of claim 1, wherein the first polypeptide is linked to a compound of interest and the polypeptide binding partner is linked to the polymer.

5. The hydrogel of claim 1 further comprising a compound of interest physically entrapped in the hydrogel or linked to the polymer.

6. The hydrogel of claim 3, 4 or 5, wherein the interaction between the first polypeptide and the polypeptide binding partner is cleaved by the addition or withdrawal of a modulating compound.

7. The hydrogel of claim 3, 4, 5 or 6, wherein the compound of interest is a drug.

8. The hydrogel of any of the preceding claims, wherein the polymer is selected from poly-vinyl-based polymers, polypeptides and polycarbohydrates.

9. The hydrogel of claim 8 wherein the polymer is selected from polyacrylamide, polyethylene glycol, poly-dimethyl-diallyl-ammonium chloride, N-(2-hydroxypropyl)-methacrylamide, fibrin, collagen, poly-L-lysine, alginate, cellulose, and starch.

10. The hydrogel of any of the preceding claims, wherein the first polypeptide and the polypeptide binding partner are selected from the pairs of a protein binding to a DNA sequence or a protein binding to a second protein.

11. The hydrogel of claim 11, wherein the first polypeptide and the polypeptide binding partner are GyrB-GyrB, FKBP-FRB, F_{M}-F_{M}, ToxT-ToxT, DHFR-DHFR, FKBP-FKBP, FKBP-Cyp, Cyp-Cyp, E-ETR, PIP-PIR, TetR-tetO, ArgR-argO, ArsR-arsO, and HucR-hucO.

12. The hydrogel of claim 11, wherein the first polypeptide and the polypeptide binding partner are one polypeptide having a tendency to dimerize by addition of withdrawal of the modulating compound.

13. The hydrogel of claim 13, wherein the polypeptide having a tendency to dimerize is GyrB, F_{M}, ToxT, DHFR, FKBP, and Cyp.

14. A system of drug delivery comprising the hydrogel of claim 1, wherein either the first polypeptide or the polypeptide binding partner are linked to the polymer, and the corresponding polypeptide binding partner or the first polypeptide, respectively, are linked to a drug, and further comprising a compound cleaving the interaction between the first polypeptide and the polypeptide binding partner.

15. A system of drug delivery comprising the hydrogel of claim 1, wherein the first polypeptide and/or the polypeptide binding partner are linked to the polymer and a drug is physically entrapped in the hydrogel structure or bound to the polymer stabilized by the interaction between polypeptide and the polypeptide binding partner, and further comprising a compound cleaving the interaction between the first polypeptide and the polypeptide binding partner thereby loosening the hydrogel structure to set free said drug.
